# EUROPEAN PATENT APPLICATION

(11) **EP 0 611 592 A2**
(43) Date of publication of application: **24.08.1994**
(21) Application number: 94101277.5
(22) Date of filing: 28.01.1994
(51) Int. Cl.: B01D 67/00, B01D 69/00, B01J 20/32, B01D 15/08

(54) **Membranes for use in affinity separation and methods for activating membranes**

(30) Priority: 11.02.1993 GB 9302754
(71) Applicant: Pall Corporation, East Hills, New York 11548 (US)
(72) Inventor: Troth, Harvey Graham, Drayton, Portsmouth P06 2AU (GB)
(74) Representative: Geissler, Bernhard, Dr.

(57) **Abstract**

Membranes for use in affinity separation and methods of activating such membranes are provided having increased binding capacity in affinity separation processes. Porous membranes are contacted with activating agents to form linking molecules which are particularly effective in the production of biologically active membranes. Biologically active membranes are also provided, which are prepared by contacting chemically active membranes with a volume of solution containing an acceptor ligand molecule for immobilization which is close to the void volume of the membrane. After void volume loading, the membrane is dried under controlled conditions. Biologically active membranes produced in this manner are found to have a greatly increased capacity of reversibly immobilizing ligates from solution.

## Description

The present invention relates to a method for preparing an activated membrane suitable for use in affinity separation. The invention also relates to membranes obtained by the method which are capable of binding acceptor molecules. The invention further relates to a method of preparing a biologically active membrane having an acceptor molecule covalently attached thereto. Additionally, the invention relates to a method of extracting a mobile ligate from solution making use of the biologically active membrane.

The term "affinity separation" is used to describe a process by which biologically active substances, for example antibodies, antigenic substances, immunoglobulins, proteinaceous substances or other active materials capable of biospecific complex formation are extracted or isolated from a fluid. The procedure involves passing the fluid through a porous material, for example porous beads or a porous membrane, which has acceptor molecules bound thereto, which are capable of immobilizing the biologically active substance to be separated. Such active substances are referred to herein as "ligates". The acceptor molecules representing the pair molecule for complex formation are, of course, also biologically active. To distinguish these molecules from the mobile ligates, they are referred to herein as "ligands" or "acceptor ligand molecules".

A number of membrane materials have been suggested for use in affinity separation. US 4,340,479 discloses a membrane made of Nylon 66. The membrane comprises amine and carboxylic groups on its surface which are capable of binding an acceptor ligand molecule or are capable of binding with a linking molecule which in turn reacts with the acceptor molecule. The term "surface" or "membrane surface" as used herein is intended to refer to both the external surface of the membrane exposed to view as well as the internal surfaces of the pores of the membrane.

The UK Patent Application GB-A 2 115 344 discloses a polyamide membrane containing carboxyl groups on its surface and the European Patent Application EP-A-272 841 and EP-A-272 842 discloses a surface modified porous membrane comprising hydroxyl groups. The functional groups on the surfaces of such membranes, for example amines, hydroxyl or carboxyl groups, can be activated with an activating agent providing a site on the membrane surface which is capable of binding an acceptor molecule. The UK Patent Application GB-A 2 163 434 discloses for example the activation of the mentioned surface modified Nylon 66 membrane with trichloro-s-triazine or carbodiimide as the activating agent.

The reaction of the activating agent with the functional groups or reactive moieties on a membrane surface leaves a residue molecule attached to the surface which is referred to herein as the linking agent or linking molecule. As indicated above, various membranes and various activating agents have been suggested in the art which are at least partly effective in immobilizing biologically active materials or ligates.

Despite advances in this field, improvements in the membranes used for affinity separation are desirable. One aspect would be to attach greater amounts of acceptor molecule to the membrane by means of the linking agent. It would be desirable to either attach more acceptor molecule or to bind it to the membrane surface in a more effective manner, thereby providing a higher separation capacity of the ligate. A need therefore exists for improved membrane materials having a better capability of attaching acceptor ligand molecules as well as methods for attaching such molecules to an activated membrane, i.e. a membrane having been reacted with the activating agent to form a linking molecule.

An object of the present invention is therefore to provide an activated membrane and a method of preparing an activated membrane by which the linking molecule formed on the surface thereof is capable of efficiently binding with an acceptor molecule. A further object of the invention is to provide a biologically active membrane and a method of preparing same, comprising acceptor molecules in a form and in an amount which allows increased ligate separation capacity. A still further object of the invention is to provide an improved method for more efficiently recovering a ligate from a biological solution. A still further object of the invention is to provide a membrane system which can be used at a higher flow rate than existing separation methods.

In accordance with the present invention, a method of preparing an activated membrane and the membrane obtained by the method are provided as defined in the claims. The method includes providing a porous membrane having reactive moieties on the surface thereof. Preferred moieties include functional groups selected from hydroxyl, carboxyl and amine groups. The porous membrane is then contacted with an activating agent capable of reacting with the moieties to form a linking molecule. The linking molecule will generally be a residue of the reaction between the activating agent and the moiety. The activating agent is selected from the group of acryloyl chloride, acetyl chloride, thionyl chloride and poly(acryloyl chloride).

The preferred activating agent is acryloyl chloride which when reacted with the moiety provides a linking molecule having an unsaturated carbon double-bond. The double-bond is conducive to reaction with an amine group of an acceptor molecule. For example when the acceptor molecule is a proteinaceous substance, the active amine sites of the protein effectively bind with the unsaturated double-bond.

In a further embodiment of the method, the membrane is further reacted with a second activating agent to produce a residue or a second linking molecule having a functional amine group. This membrane is preferably then contacted with a third activating agent which forms a third linking molecule having one or more functional aldehyde groups or hydroxyl groups. With this method, a multiplication effect can be achieved in that several functional groups capable of attaching an acceptor molecule are created from one moiety site.

A membrane is also provided by the invention comprising a linking molecule attached to the surface of the pores of a porous membrane, where the linking molecule is derived from reaction of an activating agent with the surface of the membrane, the activating agent selected from the group of acryloyl chloride, acetyl chloride, thionyl chloride and poly(acryloyl chloride) or two or more thereof. Further provided by the invention is a membrane comprising a linking molecule obtainable by reacting one or more groups selected from acryloyl anhydride, acid chloride and poly(acryloyl chloride) groups with one or more activating agents selected from the group of ammonia, substituted or unsubstituted hydrocarbyl polyamine with 2 to 12 amine groups, preferably alkylene polyamines. The activating agent is preferably alkylene diamine, more preferably a C₄-C₈ diamine, most preferably hexamethylene diamine or polyallylamine.

Further in accordance with the invention, a method of preparing a biologically active membrane is provided as defined in the claims. The method comprises providing an activated membrane having a linking molecule on the surface thereof, which is capable of binding with an acceptor ligand molecule and immobilizing it. The activated membrane described above is useful in this method, however, any suitable membrane activated to have a linking molecule bound to its surface may be employed. The method contemplates contacting the activated membrane with a specific volume of solution containing the acceptor ligand molecule. The volume of solution is in the range of 50 *%* to 150 *%*, preferably 80 *%* to 120 *%* of the void volume of the membrane. Generally, the amount of acceptor molecule-containing solution will be substantially equal to the void volume of the pores of the membrane.

This procedure, compared with known methods of immersing the membrane in excess solution, provides a more efficient usage of the acceptor molecule. The membranes thus formed have a higher capacity for immobilizing ligands from a biological solution. The complex formation between the immobilized acceptor ligand molecule and the mobile ligate will normally be a biospecific one. The membranes prepared are therefore referred to herein as biologically active membranes.

In a preferred embodiment, the membrane is dried under suitable conditions. When the relative humidity of the drying environment is within the range of 10 to 65 *%*, preferably in the range of 15 to 40 *%*, the formed biological membrane is very efficient in ligate immobilization.

The present invention also provides a biologically active membrane, the membrane having an acceptor or ligand molecule, selected from the group of antibodies, antigenic substances, glycoproteins, Protein A, Protein G, lectins, carbohydrates, enzymes, cofactors, inhibitors, hormones, IgG class of immunoglobulins, carrier proteins, receptors, heparin, coagulation factors, histones and mimetic ligands attached thereto by a linking molecule as defined in the claims.

In accordance with a further embodiment of this invention a method or process for separating biologically separable, particularly biologically active, substances from solutions containing these, is provided. This method is defined in the claims.

Preferably a prefiltered impure solution is the starting material. These impure solutions may consist of culture supernatants in general and in particular tissue culture supernatants from which the cells etc. have been removed by prefiltration, preferably to 0.2 *µ*m.

The impure solution is applied to the separation material or device in accordance with this invention. The separation material or device contains the activated sites defined herein. Upon contact with the impure solution, biologically separable substances will be attached to those sites, thereby removing these substances from the impure solution.

Usually the impure solution contains 0.01 to 0.1, typically about 0.05 mg/ml of biologically active substance. This solution is applied to the device or membrane defined herein. For example, a filter cartridge of 5 cm diameter, 5 cm high is employed at a flow rate of preferably 0.1 to 5, more particularly about 1 l/min.. Thereby, 10 to 500 mg, typically 50 to 100 mg of biologically active material is bound to this filter medium of the cartridge. This filter medium is then washed with a phosphate buffer solution to remove impurities. The bound biologically active material is then eluted using another buffer, for example glycine/acetic acid mixture in the case of IgGs, to give a much more concentrated solution of relatively pure IgG, for example a solution with an IgG concentration of 1 mg/ml. Preferably, this more concentrated solution is then further processed to remove the biologically separable, in particular the biologically active substances to obtain a solid material.

Preferably, the device is then washed, in particular with phosphate buffer solution to make it ready for a further purification from a prefiltered impure solution. It is envisaged that this cycle may be repeated tens of times, possibly with an intermediate laundering process where the device is washed, for example, with 4M urea solution at a pH of 6 to remove unwanted contaminating protein and the like which might interfere with the separation process described above.

Thus, the preferred method involves a plurality of cycles, each comprising
- a separation period, in which the impure solution is put into contact with the separation material or device or membrane as herein defined, preferably the solution is passed through the separation material or device or membrane,
- a wash period, in which the separation material device or membrane is washed substantially free of said solution while maintaining a substantial quantity of the biologically separable substance on the separating material or device or membrane,
- an elution period, in which the biologically separable substance is removed from the separating material or device or membrane, preferably to obtain a more concentrated solution of the separable material,
- optionally, an intermediate laundering step, in which the separating material or device or membrane is subjected to a stronger cleaning operation, in particular to remove unwanted or contaminating protein and the like from the separating material or device or membrane.

Preferably 10 to 100 of these cycles are performed with the same separating material or device or membrane before it is discarded.

Further embodiments and advantages of the present invention will become apparent in the following description in conjunction with the drawings.

Figure 1 shows three reaction schemes representing preferred embodiments of the invention, where acryloyl chloride is the activating agent.

Figure 2 shows various reaction schemes involving secondary use of amines and then aldehydes to provide an activation chemistry. The last scheme involves the multiplier procedure for activating membranes.

Figure 3 shows a schematic reaction scheme for reacting poly(acryloyl chloride) with a membrane comprising OH groups.

Figure 4a shows test results relating to the aqueous stability of a membrane activated in accordance with the present invention and the rate of reaction with target molecules containing SH and NH₂ groups.

Figure 4b illustrates the use of mercapto-ethanol and ethanolamine as blocking agents for removal of unreacted or unwanted activation chemistry.

Figure 5 shows the rate of activation of a membrane with various acceptor molecules.

Figure 6 shows the effect of relative humidity on the binding capacity of a membrane for IgG.

Figure 7 shows the binding capacity of a membrane for IgG as a function of the relative humidity and as a function of the concentration of Protein A used in activating the membrane.

A preferred porous membrane, useful in the present invention, having reactive moieties on the membrane surface, is a polyamide membrane with controlled surface properties. Such membranes are either prepared in a manner such that chemical functional groups or moieties are already formed on the surface or the membranes are surface-modified with agents which will produce the functional groups in sufficient concentration. Typical membranes of this type, useful in the present invention, include a poly(hexamethylene adipamide) membrane described in the US Patent US 4,340,479 and sold under the trade name Ultipor® N 66. The membrane comprises NH₃⁺ and COO⁻ groups as the reactive or chemical moieties. Another suitable membrane is sold by Pall Corporation under the name Biodyne® C and described in the UK Patent Application GB-A-2 115 344. The membrane contains carboxyl groups, some in the form of COOH and some in the form of COO⁻. A membrane comprising hydroxyl groups is also useful in the invention, for example the membrane described in EP-A-272 841, EP-A-272 842, US 4,968,533 and sold by Pall Corporation under the name Bio-Inert®.

The membrane used in the present invention is also preferably one having a high BET surface area in the range of 200 to 600 cm²/cm², more preferably in the range of 300 to 500 cm²/cm². This high surface area is particularly desirable for the ligate recovery method of the present invention. The pore rating of the membrane is preferably in the range of 0.1 to 5 *µ*m, more preferably in the range of 0.1 to 3 *µ*m.

One property of the membranes which is preferred but not necessary for the invention is that the membrane before activation should not itself bind the acceptor molecule or mobile ligate to be extracted. If for example the acceptor molecule is Protein A or an IgG, the membrane in untreated or unactivated form should not appreciably immobilize Protein A or IgG. This is referred to as non-specific binding and has been tested with the above mentioned membranes. Rabbit IgG was loaded onto ten discs of membrane material each of 47 mm diameter giving a total of 1 gram of membrane. All three membranes had a pore size rating of 0.2µm. The IgG was loaded in phosphate buffered saline (PBS) and the immobilized IgG was eluted with 0.1 M glycine/2 *%* acetic acid solution at a pH of 3.

The amount of non-specific binding of IgG in mg per gram membrane was 11.6, 7.5 and 0 to 2 for the membranes Ultipor® Biodyne® C and Bio-Inert® respectively.

The Bio-Inert® membrane comprising hydroxyl groups gives low nonspecific binding of IgG where the best results show zero binding. Although the other membranes show higher non-specific binding, they may still be used as a starting material for the present invention. Bio-Inert® is most preferred due to its relatively low non-specific binding of proteins.

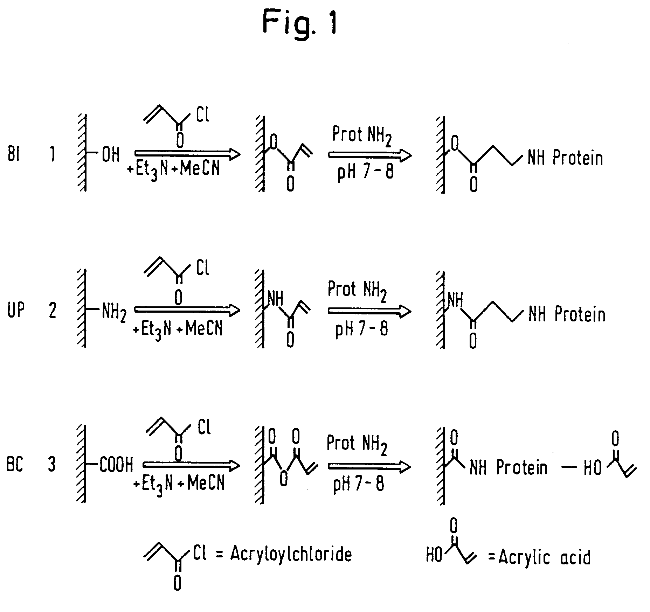

Now turning to Fig. 1, various schemes for contacting the porous membrane with an activating agent are indicated. The activating agent is acryloyl chloride. Fig. 1 also shows the further step of attaching a proteinaceous material as the acceptor molecule to the membrane once the linking molecule has been formed. This aspect of the invention, namely preparing a biologically active membrane, will be discussed further below. First, the reaction of the activating agent with the reactive moieties of the membrane surface will be discussed.

The acryloyl chloride reacts with the OH group of the Bio-Inert® (BI) membrane to form an active group or linking molecule as a residue which is similar to methyl acrylate. The linking molecule with an unsaturated double bond is capable of reacting with and binding a protein molecule as the acceptor molecule as shown in this embodiment. The reaction with the Ultipor® membrane (UP) produces a linking molecule similar to acrylamide which can also bind a protein.

The reaction of acryloyl chloride with the COOH moiety of the Biodyne® C membrane produces an anhydride as the linking molecule. As shown in Fig. 1, the anhydride can react with the protein to form a bound protein molecule and acrylic acid. Another possibility is that carboxylic acid would form on the membrane and the protein has an acrylamide residue on it. It is also possible that the protein will react with the vinyl double bond of the anhydride, which will eventually hydrolyse. These mechanisms represent possible explanations of the chemical mechanisms involved, while the invention is not thereby limited.

The activating agents are ones which preferably form a linking molecule having an unsaturated double bond as shown in Fig. 1. The activating agent is selected from a group of acryloyl chloride, acetyl chloride, thionyl chloride and poly(acryloyl chloride). The monofunctional acid chlorides have all been found to produce effective linking molecules in the form of an anhydride. The poly(acryloyl chloride) produces a linking molecule having several active sites, each capable of binding an acceptor molecule. This situation will be discussed further below.

The activated membrane of the present invention will then comprise a linking molecule attached to the pore surface of the porous membrane, the linking molecule being selected from the group of acryloyl, anhydride, acid chloride and poly(acryloyl chloride)-groups or two or more thereof.

In a further embodiment of the activation method, unreacted chemistry on the membrane surface can be removed or blocked. The unreacted or unwanted residual activation sites (for example OH groups or other chemical moieties), can be deactivated in a process referred to herein as "blocking". Two preferred reagents for this process are mercapto-ethanol and ethanolamine. Due to the unpleasant smell of mercapto-ethanol, ethanolamine is presently preferred.

In a further embodiment of the invention, the membrane already activated to comprise a linking molecule, can be further treated with a second activating agent capable of forming a second linking molecule having a functional amine group. In this embodiment the carbon double bond chemistry can be converted to amine chemistry. The membrane treated to have an amine as a functional group of the second linking molecule can be treated in a further preferred embodiment with a third activating agent to produce a third linking molecule which can have at least one functional aldehyde group. In this manner, a linking molecule can be formed on the surface of the membrane which has more than one site capable of reacting with the acceptor molecules. This embodiment of the method can be referred to as spacer chemistry in the case of hexamethylene diamine and multiplier chemistry in the case of polyallylamine.

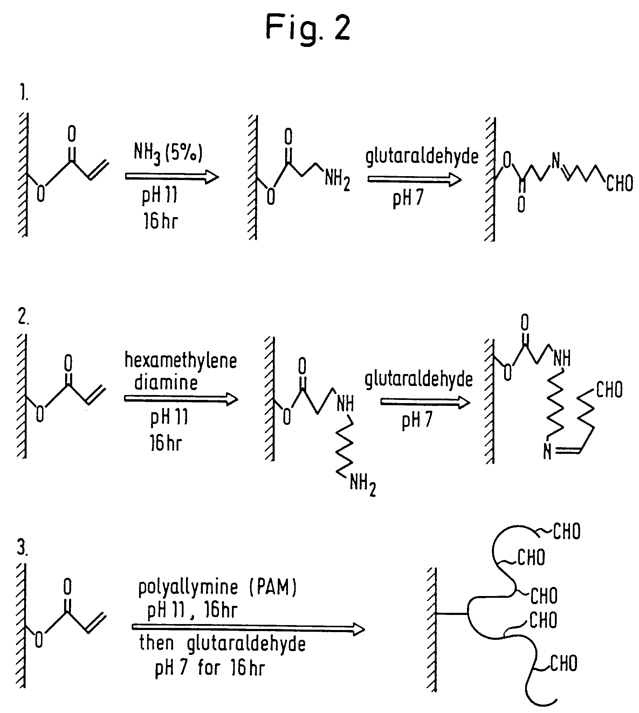

Scheme 3 of Fig. 2 shows embodiments of the reaction schemes related to multiplier chemistry. In general, the porous membrane in the first step has already been activated with acryloyl chloride to form a linking molecule having an unsaturated double bond. As shown in Fig. 2, this first linking molecule is contacted with a second activating agent which is preferably selected from the group of ammonia, substituted or unsubstituted hydrocarbyl polyamine with 2 - 12 amine groups, preferably alkane diamine. The preferred second activating agent is a C₄ - C₈ diamine most preferably hexamethylene diamine or polyallylamine Depending on the second activating agent, a second linking molecule is formed having one or more functional amine groups.

The preferred membrane, after contacting with a second activating agent, comprises a linking molecule attached to the surface of the porous membrane, the linking molecule being selected from the group of molecules obtainable by reacting one or more groups selected from acryloyl-, anhydride, acid chloride and poly(acryloyl chloride)-groups, with one or more activating agents selected from the group of ammonia, substituted or unsubstituted hydrocarbyl polyamine with 2 - 12 amine groups, preferably alkane diamine, more preferably C₄-C₈ diamine, most preferably hexamethylene diamine or polyallylamine.

The so formed membrane in a further preferred embodiment is reacted with a third activating agent preferably selected from the group of glutaraldehyde and polyacrylolein to form at least one functional aIdehyde group.

As shown in the schemes 1 and 2 of Fig. 2, glutaraldehyde is used as the third activating agent. The third linking molecule has an aldehyde group capable of subsequently binding an acceptor molecule. These embodiments are referred to as spacer chemistry.

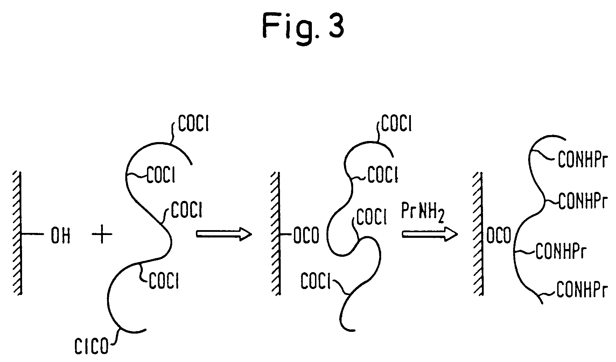

Multiplier chemistry is indicated in the reaction scheme 3 shown in Fig. 3. In this embodiment, a hydroxyl moiety, for example on a Bio-Inert® membrane, is reacted with poly (acryloyl chloride) as the activating agent. The formed linking molecule has multiple COCl sites capable of binding an acceptor molecule. In the embodiment of Fig. 3, one OH group can be activated to provide several sites for acceptor molecule immobilization.

The described membranes comprising a linking molecule formed by reaction of an activating agent with a moiety on the surface of the membrane are sometimes referred to as "chemically" active membranes. Biologically active membranes can be prepared from such chemically active membranes by attaching an acceptor molecule thereto.

According to the present invention, a general method of preparing a biologically active membrane is provided where the "chemically active" or simply "activated membrane" can be any suitable membrane comprising a linking molecule capable of binding an acceptor molecule. Particularly suited membranes are those described above, however other activated membranes are known in the art and are useful in this aspect of the invention.

The activated membrane is contacted with a volume of solution containing the acceptor molecule, where the volume is in the range of 50 *%* to 150 *%* of the void volume of the membrane. Preferably the acceptor molecule-containing solution has a volume in the range of 80 *%* to 120 *%* of the void volume of the membrane.

Contacting the void volume of the membrane with nearly the same volume of solution containing the acceptor molecule has been found to be a particularly effective procedure for loading the membrane with a biological substance or acceptor molecule. It has also been found that the drying procedure after void volume loading is of importance. It is preferred that after contact with the void volume of solution, the membrane be dried slowly at ambient temperature. Preferably the membrane is dried for 8 to 20 hours.

The acceptor ligand molecule is preferably one selected from the group consisting of antibodies, antigenic substances, glycoproteins Protein A, Protein G, lectins, carbohydrates, enzymes, cofactors, inhibitors, hormones, IgG, class of immunoglobulins, carrier proteins, receptors, heparin, coagulation factors and histones. A particularly preferred acceptor molecule is Protein A.

When the acceptor molecule is Protein A, it is also preferred that the solution be an aqueous solution and have a Protein A concentration in the range of 1 to 10 mg/ml, more preferably in the range of 2 to 6 mg/ml water.

In a further embodiment of the method, drying of the membrane after contacting with the acceptor molecule-containing solution is carried out under suitable conditions. The drying environment should have a relative humidity in the range of 10 to 65 *%*, more preferably in the range of 15 to 40 *%*. This drying is preferably carried out at temperatures in the range of 20 to 60 °C, more preferably at a temperature in the range of 25 °C to 40 °C. In this manner, the bioactivity of the acceptor molecule will under normal conditions not be destroyed. The fact that the relative humidity of the drying chamber plays a role in the efficiency of acceptor molecule attachment is a further indication that the hydration of the membrane and/or acceptor molecule can be involved in the attachment process. However, the present method of preparing a biologically active membrane is not limited to a given explanation of the mechanism actually involved.

Another biologically active membrane of the invention is a membrane having an acceptor molecule, selected from the group of antibodies, antigenic substances, glycoproteins Protein A, Protein G, lectins, carbohydrates, enzymes, cofactors, inhibitors, hormones, IgG class of immunoglobulins, carrier proteins receptors, heparin, coagulation factors and histones, attached thereto by a linking molecule defined as follows. The linking molecule is one derived by reaction of the membrane surface with an activating agents selected from the group of acryloyl chloride, acetyl chloride, thionyl chloride and poly(acryloyl chloride) or two or more thereof. The linking molecule can also be selected from the group of molecules obtainable by reacting one or more groups selected of acryloyl, anhydride, acid chloride and poly(acryloyl chloride)-groups with one or more activating agents selected from the group of ammonia, substituted or unsubstituted hydrocarbyl polyamine with 2 - 12 amine groups. The preferred activating agent is an alkane diamine, more preferably a C₄-C₈ diamine, most preferably hexamethylene diamine or polyallylamine.

The biologically active membrane of the present invention is particularly suitable for the separation of a mobile ligate from solution. A further aspect of the invention provides a method for recovering a ligate, wherein a ligate-containing solution is passed through the present biologically active membrane. The ligate to be extracted is reversibly bound to the immobilized acceptor molecule in an efficient manner. After binding, the bound ligate can be optionally removed from the membrane by elution. Generally, the reversibly bound ligate is removed by elution, however it is also possible to determine the presence and amount of ligand, for example by employing a labeling technique for the bio-specific complex formed, e.g. a radiotracer.

The following examples are intended to illustrate the advantages of the invention without limiting the invention to the embodiments disclosed therein.

To demonstrate the effect of the chemically active and biologically active membranes of the present invention, a dye test is employed in some of the examples as an alternative to attaching a acceptor ligand molecule to the membrane and then using the biologically active membrane to separate a ligate. The latter procedure is tedious and also expensive, especially when Protein A is used as the acceptor molecule.

The dye test is based on the consideration that the amount of dye attached to a chemically activated membrane will correlate with the amount of immobilized acceptor molecule which would otherwise have been attached. The amount of dye adsorbed or bound to the membrane will determine the depth of color of the resultant membrane. The color depth can be measured at least qualitatively by a densitometer. A Gretag D 182 densitometer was used in the following examples. The device measures light absorbance of a given sample, where it was found that the absorbance factor or G-factor measured by the densitometer is linearly proportional to the amount of dye adsorbed on the membrane.

Although this procedure is qualitative, ratios can be used quantitatively. For example if the absorbance or G-factor for one sample is 0.6, while the G-factor for another sample is 1.2, the activity of the latter membrane, or by extrapolation, the capacity of the latter membrane to bind acceptor molecules, is 100 *%* greater. The absorbance values were normalized using a wet non-activated sample. The absorbance is then given as the measured value of a wet activated sample, normalized to a wet non-activated sample using the magenta filter in the instrument.

Absorbance values or G-factors of greater than 0.5 were arbitrarily classified as being well activated, while values of up to 1.0 and more were observed and considered to be highly active.

### Example I

This example demonstrates the use of acryloyl chloride as the activating agent to react with the OH-groups of a Bio-Inert® membrane to form an ester as the linking agent having an unsaturated double bond. In this example, the biological activity of the produced membrane is demonstrated by attaching Protein A as the acceptor molecule to the membrane and then reversibly immobilizing rabbit IgG as the ligate in an affinity separation procedure.

Bio-Inert® membrane material with a pore rating of 0.2 *µ*m was provided in the form of 15 discs. The membrane material was activated by contacting the membrane with a solution comprising 0.5 ml acryloyl chloride, 0.5 ml triethylamine in 50 ml acetonitrile (MeCN) for 3 hours. The discs were then washed in MeCN and dried. The G-factor was 0.9. Twelve of the discs were treated with 2 ml of Protein A solution having a concentration of 10 mg/ml such that the discs were slightly oversaturated with solution. Twelve discs would be expected to imbibe 1.8 ml of solution to fill the void space of the pores of the material. The discs were allowed to react overnight at room temperature without drying out. To distinguish this example with later examples, this procedure is referred to as "immersion" loading conditions.

At this stage the G-factor was 0.6. The residual activity of the membrane was blocked with 1% ethanolamine pH of 9.5 at 37°C for 3 hours. The resultant G-factor was 0.02. The discs were then treated with 4M urea at a pH of 8 to remove loosely bond Protein A and were then washed in PBS.

Ten of the discs representing one gram of membrane material were mounted in a disc holder having a diameter of 47 mm. The set up included a suitable pump, pressure gauge and UV spectrophotometer such that solutions containing a proteinaceous material could be pumped through the cell and the passage of a protein detected by absorbance at the wavelength of 278 nm.

A solution comprising only phosphate buffered saline was first passed through the cell at a flow rate of 5ml/min. to establish a baseline on the spectrophotometer. This was followed by passing at 5ml/min. a solution of 10 ml of rabbit IgG in PBS at a concentration of 1mg/ml. Part of the rabbit IgG was immobilized and a part was passed through the membrane material. If the spectrophotometer trace revealed that no IgG passed through the membrane, a further 10ml of the above solution was passed through the membrane medium. The above process can be repeated until IgG is observed coming through the filter. The amount of IgG adsorbed before breakthrough can then be determined and is commonly called "the breakthrough capacity" of the disc set.

At this point, 100ml of PBS solution was flowed through the discs to reestablish a baseline. This later step is important for impure samples for the removal of non-immobilized impurities.

The rabbit IgG was eluted with a solution 0.1 M glycine and 2 *%* acetic acid at pH 3. It was found that 4 mg of IgG was reversibly immobilized per 1g of membrane. This was determined in two ways. Firstly, the amount of IgG in the elution peak was determined by measuring its absorption at 278 nm versus the baseline for PBS. A second method is to use the area under the peaks in the UV trace. A quantity of rabbit IgG, typically 10 mg in 10 ml PBS was passed through the device without the filter medium being present. The area under the UV trace is then known to be 10 mg. In this manner it was found that 6 mg of IgG passed through the 10 filter discs and 4 mg was subsequently eluted. Thus a mass balance was a achieved.

Of course the function of the pair of components used in the biospecific complex formation could be reversed. For example, rabbit IgG could be bound to the membrane and used to purify Protein A from crude biological extracts. The eluting agent would again be glycine/acetic acid.

### Example II

This example demonstrates the activation of membranes having various reactive moieties, again using acryloyl chloride as the activating agent. The membranes used were Bio-Inert® (BI) containing OH-groups, Biodyne® C (BC) comprising COOH-groups and Ultipor® (UP) having a surface rich in NH3⁺ and COO⁻ groups. All membranes had a pore size rating of 0.2 *µ*m. Dry discs of 47 mm diameter were treated in a 100 ml solution of acetonitrile further containing 0.5 ml acryloyl chloride and 0.5 ml triethylamine for 2 hours. The samples were washed in acetonitrile, then dried and subjected to the dye test. The reaction schemes involved are those given in Fig. 1, with the dye taking the place of the acceptor molecule.

The dye test gave absorbance factors of 0.60, 0.57 and 0.25 for the membranes Bio-Inert® (BI), Biodyne® C (BC) and Ultipor® (UP), respectively. The BI and BC membranes are well activated, while UP although less strong, can still be considered to be active.

### Example III

This example demonstrates the level of activation imparted to a membrane versus the time of activation, i.e. the time of contacting the solution containing the activating agent with the membrane. The experimental conditions are those of Example II Bio-Inert® filter membranes having a pore size rating of 0.2 *µ*m and 3 *µ*m were employed. The absorbance factors in the dye test were measured as a function of time of activation.

The 3 *µ*m membrane reached an absorbance factor of 0.5 after 5 hours. The Bio-Inert® membrane with the smaller pore rating of 0.2 *µ*m reached a higher level of activation with an absorbance of 1.3 after 16 hours.

The data show that the smaller pore membrane takes longer to activate, however, the level of activation is much higher than the 3 *µ*m membrane. It should also be pointed out that the 0.2 *µ*m membrane has a larger BET surface area.

### Example IV

This example illustrates the reactivity of the chemically active membranes of the present invention. The properties of a Bio-Inert® membrane activated with acryloyl chloride were investigated. The membrane had a pore rating of 0.2 *µ*m and the dry BI discs were activated for 2 hours to give an activation level corresponding to an G-factor of 0.55.

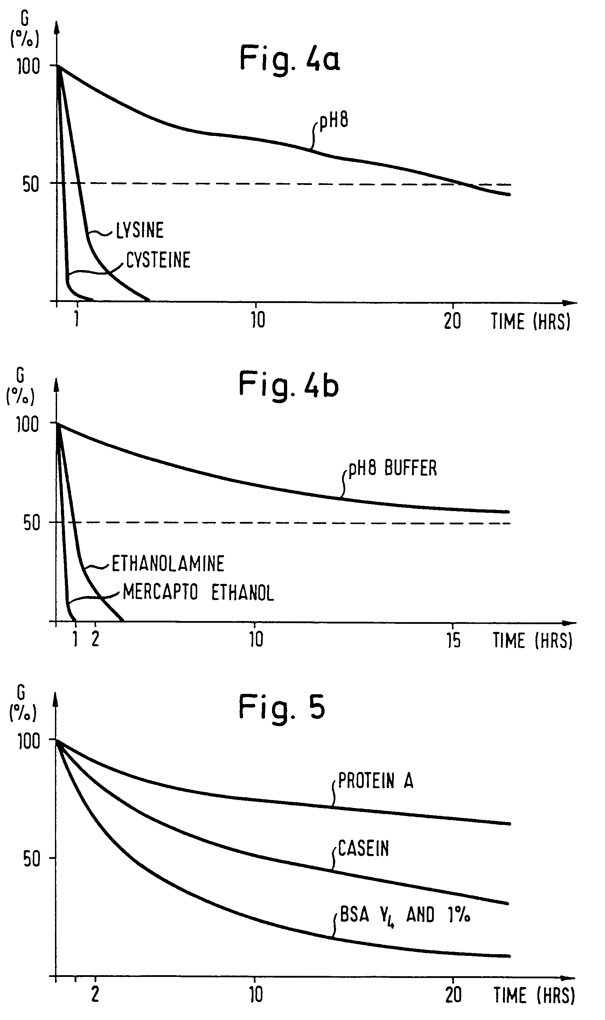

The activated membrane was then contacted with various compounds expected to bond to and deactivate the linking molecules. The discs were treated with cysteine, lysine and water, all at a pH of 8 in PBS. The absorbance of the samples were measured as a function of time indicating the rate of deactivation. Fig. 4a shows the loss of activation given as percent reduction of the absorbance G.

Cysteine reacts rapidly and has half-reacted in a few minutes. Lysine reacts more slowly and requires about 50 minutes to react with 50 *%* of the active groups or linking molecules on the membrane. The PBS buffer having a pH of 8 reacts with 50 *%* of the linking molecules only after 20 hours. The chemistry is therefore relatively stable in water and weakly alkaline buffers, while retaining reasonable activity towards amines.

### Example V

This example illustrates the removal of unreacted or undesired residual activation chemistry. A Bio-Inert® membrane was activated with acryloyl chloride as in Example IV The membrane was then reacted with 1 *%* mercapto-ethanol, 1 *%* ethanolamine both in a buffer of pH 8 and the third solution was simply a buffer of pH 8. All dissolved in the presence of 3 M NaCl.

The results are shown in Fig. 4b. A rapid reaction was found with mercapto-ethanol similar to that observed in Fig. 4a for cysteine. Ethanolamine reacted at a similar rate to lysine. The results show that both mercapto-ethanol and ethanolamine can effectively be used as blocking agents to remove undesired activation, for example after a membrane has been biologically activated.

### Example VI

The above sample treated with ethanolamine was then further tested using rabbit IgG for nonspecific binding. The starting sample of BioInert® before the present treatment had a nonspecific binding level of 2 mg IgG per gram membrane. After treatment with acryloyl chloride and ethanolamine, as outlined above, the nonspecific binding level was reduced to 0.5 mg IgG per gram membrane. The test also shows why good specific reversible binding is obtained, i.e. once the active sites are blocked, no IgG is lost via nonspecific binding.

A further test was performed to observe the affect of moisture vapor on the Bio-Inert® membrane activated with acryloyl chloride. Samples were placed in a polyethylene bag and left on the laboratory bench at a relative humidity of 50*%* for one week. The activity had dropped to 66 *%* of the initial value.

All of these tests demonstrate that the activation chemistry of the membranes produced by the present method is relatively stable in water and damp atmospheres and is much better than observed with comparable chemistries, such as activation with trichlorotriazine, FMP and cyanogen bromide.

### Example VII

This example demonstrates the preparation of a biologically active membrane from a Bio-Inert® membrane activated with acryloyl chloride. The proteins as acceptor molecules chosen were bovine serum albumin (BSA), Protein A and casein. The Bio-Inert® membrane had a pore size rating of 3 *µ*m. Protein A (10 mg/ml) in a 3 M NaCl solution at a pH of 8, in an amount of 0.2ml was applied to each disc (47mm in diameter), Casein was also applied at a concentration of 1*%* in NaCl solution at a pH of 6.75. BSA in a 1*%* and 1/4*%* solution at a pH of 8 was contacted with the discs of Bio-Inert® material. The rate at which the membranes became biologically active, ie. the rate at which the acceptor molecule was attached to the membrane, is indicated by a decrease in the absorbance G. The results are illustrated in Fig. 5.

The decrease in absorbance G at a given time reflects the rate at which the respective protein has been attached to the membrane. Little difference is found between the membranes loaded with 1/4*%* BSA versus 1*%* BSA This may be due to the very strong absorption of the protein onto the activated membrane surface. The data indicates that some proteins absorb or attach much more readily than others.

The results of Fig. 5 also indicate that, for example, in the case of Protein A, the membrane would still have sites available for reaction even after 20 hours. This remaining activity of the membrane can be destroyed or blocked when desired. Reagents useful for deactivating such remaining sites are mercapto-ethanol and ethanolamine tested in Example V.

### Example VIII

This example demonstrates activation of various membranes with acetyl chloride as the activating agent. Dry discs of 47mm diameter and having a pore rating of 0.2 µm were contacted with 0.5ml acetyl chloride and 0.5ml triethylamine in 100ml acetonitrile for 2 hours. The samples were washed in acetonitrile, dried and subjected to the dye test. The absorbance factors of the samples were found to be 0.75, 0.2 and 0.15 for the membranes Biodyne® C, Bio-Inert® and Ultipor®, respectively. The data demonstrate that the Biodyne® C membrane comprising carboxyl groups was well activated by acetyl chloride, while the other membranes are activated but to a lesser degree.

### Example IX

This example demonstrates the use of thionyl chloride as the activating agent. Dry discs of 47mm diameter were activated by contacting with 50ml acetonitrile containing 0.5ml Et₃N and 0.5ml thionyl chloride. The solution turned green/yellow and then through to brown. The degree of activation was determined qualitatively by the dye absorbance test. The G factor for the Biodyne® C membrane was 0.95, for Bio-Inert® 0.75 and for Ultipor® 0.35. The data show good activation of all three membranes with thionyl chloride.

### Example X

The following example demonstrates activation of a membrane by an activating agent which has multiple functional groups. A Bio-Inert® membrane was employed having OH functional groups in high concentration attached to its surface. The membrane was activated with poly(acryloyl chloride).

A sample of poly(acryloyl chloride) was made by heating the following ingredients at 65°C for 6 hours: 2ml of acryloyl chloride in 50ml heptane containing 0.25g of a free radical initiator AZBN (azo-bis-isobutyronitrile). At the end of the reaction 1ml triethylamine was added and a precipitate was formed comprising the poly (acryloyl chloride)/ Et₃N complex. The monomer complex is soluble in THF, which was then removed by washing with THF. The polymer was finally dissolved in acetonitrile and employed in membrane activation.

The Bio-Inert® Biodyne® C and Ultipor® membranes were activated. The activity was estimated visually using the dye test. The Bio-Inert® and Biodyne® C membranes had approximately equivalent activity, both being better than the Ultipor® membrane.

The experiment was repeated with a sample of poly(acryloyl chloride) purchased from PolyScience Inc. with the same result. The absorbance G factors were 1.04, 1.02 and 0.4 for the membranes Bio-Inert® Biodyne® C and Ultipor® respectively.

The data indicate very good activation with a multi-functional activating agent. This example shows the advantage of the multiplier type chemistry. From 1 functional OH group on the Bio-Inert® surface, several COCl sites are formed, each of which is capable of binding an acceptor molecule.

### Example XI

The present example demonstrates an embodiment of spacer chemistry in accordance with the invention. In the present case, an acryloyl chloride activated membrane is subsequently reacted with further activating agents, namely an amine and then an aldehyde.

A Bio-Inert® membrane was first activated with acryloyl chloride according to the procedure of Example I. The membrane was then contacted with a 1*%* ammonia solution at a pH of 11 for 16 hours. This treatment was followed with contacting the membrane in glutaraldehyde at a pH of 7.

The degree of activation at each stage of the procedure was tested by dye absorbance. The initial activated Bio-Inert® membrane had a G factor of 1.1, after ammonia treatment a value of 0.02 and after glutaraldehyde a factor of 0.75.

To test the biological activity, ten discs of the resultant membrane were then contacted with Protein A as in Example 1 under immersion conditions. A reaction scheme is illustrated in part 1 of Fig. 2. The membrane stack was found to reversibly bind 5mg of rabbit IgG per 1g of membrane. The results indicate the success of the reaction scheme employing multiplier chemistry as indicated in part 1 of Fig. 2.

### Example XII

The example illustrates the multiplier chemistry of the reaction scheme shown in part 3 of Fig. 2. Again a Bio-Inert® membrane was activated with acryloyl chloride according to the procedure of Example I. The resultant membrane had an absorbance factor of 1.1 in the dye test. The membrane was then contacted with the 1*%* solution of polyallylamine (PAM) at a pH of 11 for 16 hours. The treated membrane had a G factor of 0.09. In a further step the resultant membrane was treated with a third activating agent being glutaraldehyde. A 1*%* solution of glutaraldehyde was contacted with the membrane at a pH of 7 for 16 hours. The membrane had an absorbance value of 0.55, again indicating good activity.

Samples of the membrane were also employed in an affinity separation test. Ten discs of the membrane (1 gram of membrane) were loaded with Protein A according to the immersion procedure of Example I. The membranes were found to bind 6mg IgG per gram of membrane. This result shows the success of the multiplier effect indicated in the reaction scheme of Fig. 2.

### Example XIII

The following example demonstrates the method of preparing a biologically active membrane having increased capacity for binding a ligand.

In the present example Protein A is used as the acceptor molecule and rabbit IgG as the mobile ligate to be separated. Protein A has a molecular weight of approximately 42000 while the molecular weight of IgG is approx. 160,000. Both Protein A and IgG in the hydrated form have approximately the same hydrodynamic radius. Assuming that each Protein A molecule attached to the membrane surface should immobilize or bind with one IgG molecule and if the BET surface area of a Bio-Inert® membrane with a pore rating of 0.2 *µ*m is about 400cm²/cm² and is smooth on a 100 Angstrom scale, then it can be calculated that about 6 mg of solvated Protein A should be attachable to 1 g of membrane. This amount of membrane should then theoretically be capable of immobilizing about 22 mg of IgG. This can be considered the theoretical maximum for the separation process.

Ten discs of Bio-Inert® membrane were activated with acryloyl chloride in acetonitrile solution containing Et₃N according to the method of Example I. The stack of ten membranes each of 47mm diameter represent a total of 1 gram of membrane.

The activated membranes were then contacted with a solution containing Protein A (8 mg/ml PBS) in a volume of water just sufficient to fill the voids of the membrane. In the present case the ten discs required approx. 1.5ml of Protein A solution to fill the voids. After imbibing the Protein A, the membrane was dried slowly, i.e. the discs were allowed to dry out overnight in the dry laboratory atmosphere at ambient temperature. This "slowly dried" void volume loaded membrane was then tested for its capacity to bind rabbit IgG as outlined in Example I, but offering 20 mg of IgG to the membrane. It was found that the "void volume loaded" and "slowly dried" membrane was capable of immobilizing 10mg of IgG per gram of membrane. This contrasts with the immersion (non dried) loading of the membrane in Example I, where only 4 mg IgG per gram membrane was recovered.

### Example XIV

The Examples XI and XII were repeated, but now using the void volume loading and drying out process for attaching the acceptor molecule (Protein A) to the converted aldehyde chemistry. The binding capacities for IgG increased to 15 and 19mg per gram membrane for the ammonia/PAM modified membranes described in Examples XI and XII, respectively. The maximal theoretical capacity of 22ml per gram membrane as discussed above is closely approached, indicating the significance of the loading and drying method.

### Example XV

This example indicates a further embodiment of the void volume loading/drying, where the drying out of the membrane after Protein A loading is performed under controlled conditions. The Bio-Inert® membrane with the pore rating of 0.2 *µ*m was activated as described in Example I. The 1 gram of membrane material (ten discs of 47mm diameter) were contacted with 2.0ml of solution of Protein A. This is a slight excess of solution over the void volume (1.5ml). The concentrations of the Protein A solution were 2, 4 and 8mg/ml.

After contacting with the solution, the discs were allowed to dry at room temperature (25°C) in a covered glass tank in which sulfuric acid solution had been placed to be able to adjust the relative humidity (RH) in the drying container The dishes containing sulfuric acid were placed at the bottom of the container to provide a large surface area for the acid solution to act as a drying agent. Above this was placed a perforated stainless steel sheet and on top of this a polypropylene net. The disc samples were placed individually on the net and allowed to dry overnight for 16 hours in the controlled RH atmosphere.

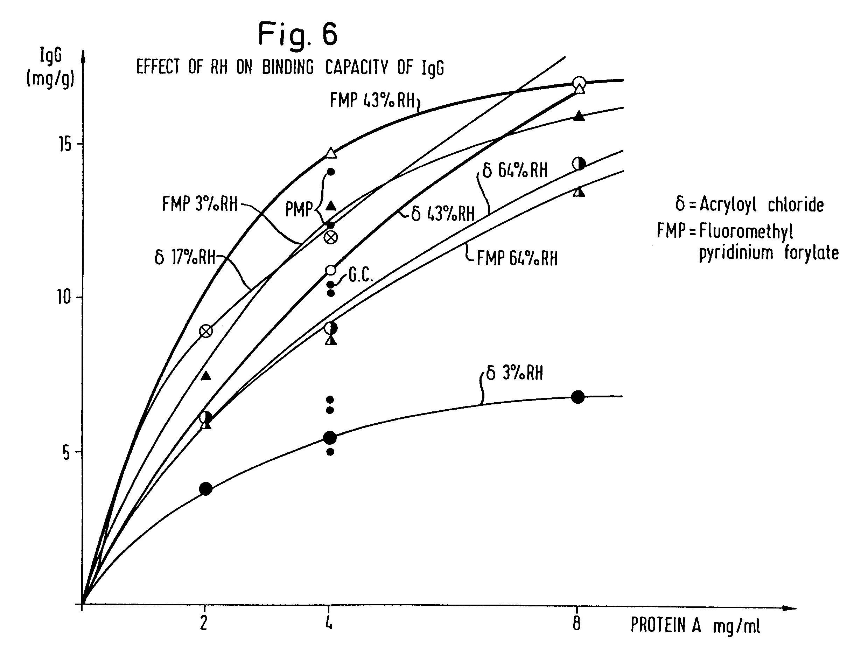

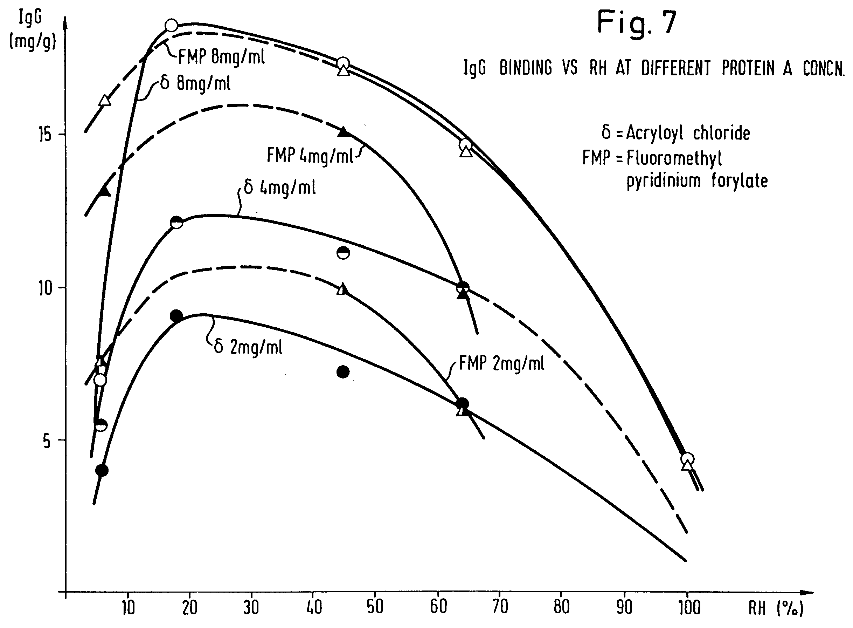

The resultant samples were then tested for their binding capacity of IgG. The results are given in Fig. 6 and 7, where it can be seen that IgG binding capacities of nearly 20 mg per gram membrane are obtained at the optimum relative humidity.

Further tests were performed using different activation chemistries. Disc samples were activated using FMP (fluoromethylpyridinium tosylate) in acetonitrile containing 0.6*%* triethylamine. These samples were washed with acetonitrile and dried according to the procedure above at different values of relative humidity. The results of the binding capacity for IgG of these samples are also shown in Fig. 6 and 7.

Fig. 7 shows that not only the acryloyl chloride but also the FMP activated membranes have a greatly increased binding capacity. Fig. 7 shows that at a relative humidity of 43*%*, both the acryloyl chloride and the FMP membranes are capable of immobilizing 17mg IgG per gram of membrane. This is for a loading solution of Protein A having a concentration of 8mg/ml. The IgG binding level approaches the theoretical maximum of approx. 22mg IgG for 1 gram of membrane for a 0.2 *µ*m membrane with a BET surface area of 400 cm²/cm². The data indicate that the loading procedure is effective for various activating agents.

Figure 8 shows the present results, however plotted as IgG binding capacity vs. relative humidity in the drying procedure. It can be seen that there is an optimum region of relative humidity for drying the discs. Good results are obtained in the range of 10 to 65*%* relative humidity, while the best results are obtained in the range of 15 to 40*%* relative humidity. The above loading procedure is referred to as the "void volume loading with controlled drying out process".

### Example XVI

The void volume loading with controlled drying out process was again tested in the present example. Duplicate sets of 10 discs having pore ratings of 0.2, 0.45, 1.2 and 3 *µ*m were employed. The membrane sets were activated according to Example I, where 0.2 ml of Protein A solution in PBS (8 mg/ml) was added to each disc such that 1 gram of disc material was contacted with 2 ml of the Protein A solution (8 mg/ml).

The discs were dried out in a controlled manner overnight as described in Example XV. The controlled humidity was provided by 60 ml of concentrated H₂SO₄ mixed with 100 ml water to give a relative humidity of 15 - 20 *%* The dry discs were then treated with 1 *%* ethanolamine at a pH of 9.5 for 3 hours at 37°C to "block" or deactivate the remaining sites of the disc and to preserve the low non-specific binding thereof.

The binding capacities are shown in the following table

| Rating | Rabbit IgG Binding; mg/g Membrane | | |
|---|---|---|---|
| *µ*m | Sample 1 | Sample 2 | Average |
| 0.2 | 18.5 | 19.0 | 18.75 |
| 0.45 | 18.0 | 17.5 | 17.75 |
| 1.2 | 14.0 | 12.5 | 13.25 |
| 3.0 | 9.5 | 10.4 | 10.0 |

The surprisingly good results demonstrate the superior binding capacities obtainable under controlled drying conditions over those obtainable under immersion conditions.

### Example XVII

The above examples relate to discs dried out individually in a controlled RH environment. The present Example relates to the loading of membrane material provided in the form of a multilayered, pleated medium. Such material will usually be of generally cylindrical shape and can be endcapped and provided in the form of a filter cartridge. In the present Example, a medium comprising 16 layers wrapped spirally and subsequently pleated is employed. The cylindrical medium has 16 pleats and is about 5 cm high. The total membrane surface area is about 3000 cm² and the weight of the membrane was about 17 grams. The area of the outer layer of the cylindrical medium was 187 cm² and it is this area together with the 16 layers and the pore rating which determine the pressure drop across the medium or cartridge.

The cartridge was activated for 3 hours with 1.5 ml acryloyl chloride and 1.5 ml triethylamine in 150 ml acetonitrile. It was then washed with 150 ml acetonitrile which is forced through the pores by means of air pressure. The cartridge was then dried to constant weight in a vacuum oven at 80°C for about 1 hour.

Two different Protein A solutions (4 mg/ml and 8 mg/ml) in a volume of 35 ml were then used to load the membrane material of the cartridge from the inside, while rotating it to give an even coating but still under void volume loading conditions. The cartridge contains 16 layers of media and it was considered that drying out under the conditions used for discs, which could of course be used, would be too slow to achieve optimum results. Therefore a faster drying out procedure was successfully used to enhance the properties of the cartridge. This involved substantially drying out of the cartridge in a vacuum oven at 80°C. The temperature of the cartridge was monitored using a thermocouple. Water evaporation produces a cooling effect such that the temperature of the cartridge started at 22°C and rose to 37°C over a period of 3 hrs when 20g of water was lost. The remaining 15g of water was removed in an oven with fresh air circulation at 37°C overnight when the cartridge became substantially dry.

After the drying out procedure, the dried cartridge was treated with 1 *%* ethanolamine at pH of 9.5 for 3 hours at 37°C. The cartridge was then washed with water followed by PBS. The binding capacity of the so-loaded cartridges to rabbit IgG was measured as described earlier, but using much larger quantities of rabbit IgG corresponding to the enhanced capacity of the cartridges. The results are shown on the following table.

| Cartridge Rating | Δp 1 liter/min | Breakthrough Capacity (mg) for Rabbit IgG at a Protein A Loading of | |
|---|---|---|---|
| µm | bar | 4 mg/ml | 8 mg/ml |
| 0.2 | 7 | 128 | 230 |
| 0.45 | 3.5 | 120 | 210 |
| 1.2 | 1.6 | 110 | 140 |
| 3.0 | 1 | 90 | 94 |

As can be seen from the high breakthrough values, cartridges containing multilayered, pleated membrane material, biologically activated in accordance with the present invention, will provide greatly enhanced amounts of ligate in affinity separation processes. The best results are obtained for small pore rating and high concentrations in Protein A loading, however the conditions of the particular case will dictate the economy of using higher or lower ligand concentration.

## Claims

1. Method of preparing an activated membrane comprising the steps of:
a) providing a porous membrane, having reactive moieties on the surface thereof, and
b) contacting the porous membrane with an activating agent capable of reacting with said moieties to form a linking molecule, the activating agent being selected from the group of acryloyl chloride, acetyl chloride, thionyl chloride, and poly(acryloyl chloride).

2. Method of claim 1, wherein the reactive moieties are selected from the group of hydroxyl, carboxyl and amine groups.

3. Method of claim 1 or 2, wherein the activating agent is acryloyl chloride and the reactive moieties on the surface of the porous membrane are hydroxyl groups.

4. Method of claim 1, 2 or 3, further comprising after step b), the step of:
c) contacting the membrane with a second activating agent capable of reacting with the formed linking molecule of step (b) to produce a second linking molecule having a functional amine group, and preferably
d) contacting the membrane of step (c) with a third activating agent capable of reacting with the second linking molecule to produce a third linking molecule having at least one functional aldehyde group or at least one functional hydroxyl group.

5. Method of claim 4, wherein the second activating agent is selected from the group of ammonia, substituted or unsubstituted hydrocarbyl polyamine with 2 - 12 amine groups, preferably alkane diamine, more preferably C₄-C₈ diamine, hexamethylene diamine and polyallylamine.

6. Method of claim 4 or 5, wherein the third activating agent is selected from the group of glutaraldehyde and polyacrolein to form functional aldehyde groups.

7. Method of one of the preceding claims, wherein said porous membrane is a microporous, polyamide membrane, which has been surface modified to comprise said reactive moieties, in particular wherein said polyamide is poly(hexamethylene adipamide).

8. Method of claim 7, wherein the pore rating of the polyamide membrane is in the range of 0.1 to 5 *µ*m, in particular wherein the pore rating is in the range of 0.1 to 3 *µ*m.

9. Method of one of the preceding claims, where after contacting the porous membrane with one or more activating agents, the membrane is contacted with a blocking agent, preferably mercapto-ethanol or ethanolamine.

10. A membrane comprising a linking molecule attached to the surface of a porous membrane, the linking molecules being derived from reaction of an activating agent and the surface moieties of the membrane, said activating agent selected from the group of acryloyl chloride, acetyl chloride, thionyl chloride and poly(acryloyl chloride) or two or more thereof.

11. A membrane comprising a linking molecule attached to the surface of the pores of a porous membrane, the linking molecule being selected from the group of molecules obtainable by reacting one or more groups selected from acryloyl, anhydride, acid chloride and poly(acryloyl chloride)-groups, with one or more activating agents selected from the group of ammonia, substituted or unsubstituted hydrocarbyl polyamine with 2 - 12 amine groups, preferably alkylene diamine, more preferably C₄-C₈ diamine, hexamethylene diamine and polyallylamine.

12. A membrane comprising a linking molecule capable of binding an acceptor ligand molecule, wherein the membrane has been prepared by the method of one of the claims 1 to 8.

13. Method of preparing a biologically active membrane comprising an acceptor ligand molecule capable of reacting with a ligate to form a biospecific complex, the method comprising the steps of:
a) providing an activated membrane comprising a linking molecule on the surface thereof capable of binding with and immobilizing the acceptor molecule, and
b) contacting the activated membrane with a volume of solution preferably an aqueous solution containing said acceptor ligand molecule in the range of 50 *%* to 150 *%* of the void volume of the membrane, preferably 80 *%* to 120 *%* of the void volume of the membrane.

14. Method of claim 13, wherein said acceptor molecule is selected from the group of antibodies, antigenic substances, glycoproteins, Protein A, Protein G, lectins, carbohydrates, enzymes, cofactors, inhibitors, hormones, IgG class of immunoglobulins, carrier proteins, receptors, heparin, coagulation factors, histones and mimetic ligands, in particular wherein said acceptor ligand molecule is Protein A

15. Method of claim 13 or 14, wherein the activated membrane is the membrane of claim 10 or 11 or a membrane obtained by the method of one of the claims 1 to 8.

16. Method of one of the claims 13 to 15, wherein Protein A is the acceptor molecule and said solution is an aqueous solution with a Protein A concentration in the range of 1 to 10 mg/ml, preferably in the range of 2 to 6 mg/ml.

17. Method of one of the claims 13 to 16, further comprising after the contacting step b, the step of drying the membrane under controlled conditions at ambient temperature for 8 to 20 hours.

18. Method of one of the claims 13 to 16, further comprising after the contacting step b, the step of drying in an environment of a relative humidity in the range of 10 to 65 *%*, more preferably in the range of 15 to 40 *%*, and preferably carried out at temperatures in the range of 20 to 60°C, more preferably at a temperature in the range of 25 to 40°C.

19. Method of one of the claims 13 to 18, wherein the activated membrane is provided as a multilayered, pleated structure of generally cylindrical form.

20. A biologically active membrane suitable for ligate separation, the membrane having an acceptor ligand molecule, selected from the group of antibodies, antigenic substances, glycoproteins Protein A, Protein G, lectins, carbohydrates, enzymes, cofactors, inhibitors, hormones, IgG class of immunoglobulins, carrier proteins, receptors, heparin, coagulation factors, histones and mimetic ligands attached thereto by a linking molecule as defined in claim 10 or 11.

21. A biologically active membrane suitable for ligate separation, the membrane having been prepared by the method of one of the claims 13 to 19, preferably wherein the membrane is provided as a multilayered, pleated structure of generally cylindrical form.

22. Process for separating biologically separable, more particularly biologically active substances from impure solutions containing same, said process comprising
contacting said impure solution with a product, namely a separating material or device or membrane, in accordance with one of the preceding claims, thereby separating said substance from said solution by immobilizing it at said product,
and removing said remaining impure solution from said product.

23. Process in accordance with claim 22, further comprising recovering said separated substance from said product, preferably by elution.

24. Process in accordance with claim 22 or 23, comprising,
- immobilizing said substance at said product,
- removing impure solution from said product by washing said product while maintaining a substantial quantity of said substance on said product,
- eluting said product and thereby removing substance from said product, preferably obtaining a more concentrated solution of said substance in comparison with said impure solution,
- optionally laundering said product to remove unwanted and/or contaminating proteins and other alien materials from the product.

25. Process in accordance with claims 22, 23 or 24, wherein the steps are repeated, preferably in the form of 10 to 100 cycles.

26. Process in accordance with one of claims 22 to 25, characterized by employing the conditions or respectively steps of one or more of the use claims herein.

27. Process for recovering a ligate from a solution, in particular in accordance with one of the claims 22 to 26 comprising:
a) passing a ligate-containing solution through the biologically active membrane of claim 20 or 21 or the membrane prepared by the method of one of the claims 13 to 19,
b) reversibly binding the ligate to the acceptor molecule on the surface of said biologically active membrane, and
c) optionally, eluting the reversibly bound ligate from said membrane.
